# EUROPÄISCHE PATENTSCHRIFT

(11) **EP 1 308 151 B1**
(45) Veröffentlichungstag und Bekanntmachung des Hinweises auf die Patenterteilung: **06.05.2004**
(21) Anmeldenummer: 02015306.0
(22) Anmeldetag: 10.07.2002
(51) Int. Cl.: A61K 7/135

(54) **Mittel zum Entfärben oder Blondieren von Haaren**
Agent for bleaching or increasing blondness of the hair
Composition pour decolorer ou blondir les cheveux

(30) Priorität: 31.10.2001 DE 10153686
(43) Veröffentlichungstag der Anmeldung: 07.05.2003
(73) Patentinhaber: Wella Aktiengesellschaft, 64274 Darmstadt (DE)
(72) Erfinder: Schmenger, Jürgen, 64331 Weiterstadt (DE); Hess, Gabriele, 64390 Erzhausen (DE); Lauscher, Dirk, Dr., 64342 Seeheim-Jugenheim (DE)

(56) Entgegenhaltungen:
- EP-A- 0 778 020
- EP-A- 0 882 444
- DE-A- 19 909 661
- GB-A- 859 276
- PATENT ABSTRACTS OF JAPAN vol. 1998, no. 10, 31. August 1998 (1998-08-31) & JP 10 139631 A (KOKURIYUUDOU:KK;NIPPON SHIKIZAI KOGYO KENKYUSHO:KK), 26. Mai 1998 (1998-05-26)
- PATENT ABSTRACTS OF JAPAN vol. 1997, no. 09, 30. September 1997 (1997-09-30) & JP 09 124440 A (KANEBO LTD), 13. Mai 1997 (1997-05-13)

## Beschreibung

Gegenstand der vorliegenden Erfindung ist ein vor der Anwendung mit einer wässrigen Oxidationsmittelzubereitung zu vermischendes Mittel zum Entfärben oder Blondieren (Bleichen) von Haaren, insbesondere menschlichen Haaren.

Zum Entfärben oder Blondieren von Haaren werden üblicherweise oxidierende Zubereitungen verwendet, welche durch Auflösen eines sogenannten Blondierpulvers (Pulvergemisch aus Alkalisalzen und anorganischen Persalzen, wie zum Beispiel Natrium- oder Ammoniumpersulfat) in einer wässrigen Wasserstoffperoxidlösung erhalten werden.

Die Verwendung derartiger Blondierpulver, welche notwendigerweise aus mehreren Bestandteilen zusammengesetzt sind, bringt jedoch eine Vielzahl von Nachteilen mit sich. So kommt es durch die Verwendung von Rohstoffen mit unterschiedlicher Dichte häufig während des Transportes oder der Lagerung zur Trennung der unterschiedlichen Pulverbestandteile, wobei sich die schweren Pulverbestandteile im unteren Teil und die leichteren Pulverbestandteile im oberen Teil des Behältnisses ansammeln. Diese Entmischung hat zur Folge, dass die gleiche Pulvermenge je nach ihrem Entnahmeort eine unterschiedliche chemische Zusammensetzung und somit auch eine unterschiedliche Blondierwirkung aufweisen kann. Um dieser Entmischung entgegenzuwirken, ist es erforderlich, vor jeder Pulverentnahme das Pulver gründlich zu schütteln, was der Verwender in der Regel jedoch nicht tut. Eine Entmischung kann auch durch den Einsatz von Pulvergemischen mit sehr kleiner Korngröße verhindert werden. Dies hat jedoch den Nachteil, daß solche Pulvergemische - insbesondere beim Öffnen der Behältnisse, bei der Entnahme des Pulvers oder beim Vermischen mit der Wasserstoffperoxidlösung - zu einer starken Staubentwicklung neigen, was zu einer Reizung der Atmungsorgane führen kann. Weiterhin besitzen derartige Pulvergemische aufgrund der geringen Korngröße eine große Oberfläche, wodurch eine Aufnahme von Feuchigkeit beim Öffnen und Schließen des Behältnisses und damit eine Verringerung der Blondierwirkung infolge der Desaktivierung des Sauerstoffträgers begünstigt wird.

Die Zubereitung der gebrauchsfertigen Mischung erfolgt zum einen durch Verrühren der Bestandteile in einer Schale, zum anderen durch Vermischen in einer Anschüttelflasche, wobei insbesondere das Anschütteln oftmals mit einer lästigen Staubentwicklung beim Einfüllen der Komponenten in die Schüttelflasche verbunden ist.

Es wurden bereits zahlreiche Versuche unternommen, dieses Problem zu lösen. So wird in der DE-OS 40 26 235 vorgeschlagen, anstelle eines Blondierpulvers eine Mischung, bestehend aus einem Granulat der anorganischen Persulfate und einem Granulat der übrigen Bestandteile des Blondiermittels, zu verwenden. Hierdurch kann zwar das Problem der Staubentwicklung behoben werden, das Problem der Entmischung kann auf diesem Wege jedoch nicht gelöst werden, da es technisch äußerst schwierig ist, Einzelgranulate mit identischer und konstanter Korngröße oder Schüttgewicht herzustellen. Weiterhin kann es aufgrund der unterschiedlichen Löslichkeit der einzelnen Granulate zu einer Beeinträchtigung der Blondierwirkung kommen. Es erscheint zudem auch aus ökonomischen Gesichtspunkten wenig sinnvoll, anstelle eines einzelnen Granulates eine Mischung aus mehreren Granulaten herzustellen. In der EP-PS 0 560 088 wird ein pulverförmiges Mittel zum Blondieren von Haaren beschrieben, bei dem zur Verhinderung der Staubbildung ein Öl oder flüssiges Wachs zugesetzt wird. Aber auch hierdurch kann eine völlige Staubfreiheit nicht erreicht werden. Zudem ergibt sich durch den Wassergehalt der eingesetzten pulverförmigen Rohstoffe und die kompakte Pulverform eine Desaktivierung der Sauerstoffträger, wodurch das Produkt instabil wird und seine Bleichwirkung verliert. Weiterhin sind derartige Blondiermittel aufgrund ihres spezifischen Gewichtes und ihres hydrophoben Charakters für die Verwendung in der Auftrageflasche ungeeignet, da das Pulver in der Wasserstoffperoxidlösung nach unten sinkt und nicht ausreichend benetzt wird, wodurch eine inhomogene Mischung mit einem hohen Anteil an ungelöstem Pulver erhalten wird, durch die die Austrittsdüse der Auftrageflasche verstopft wird. Der Zusatz von Tensiden zur Verbesserung der Löslichkeit des Pulvers ist ebenfalls problematisch, da hierdurch die Lagerfähigkeit des Pulvers beeinträchtigt wird. In der DE-OS 38 14 356 sowie der US-PS 4 170 637 werden breiartige Zweikomponenten-Zubereitungen zur Anfertigung einer auftragefähigen, breiartigen Zubereitung zum Bleichen von Humanhaaren beschrieben, bei denen die pulverförmigen Bestandteile in eine hydrophobe Basis, bestehend aus Ölen und Wachsen, eingearbeitet sind, so daß eine Paste resultiert. Diese Suspensionen haben den Nachteil, dass sie leicht zu Phasentrennungen neigen, die sich als Ölabscheidung bemerkbar machen. Um dieses zu verhindern, wird ein absorbierendes Mittel wie zum Beispiel disperse Kieselsäure zugesetzt, wodurch wiederum die Paste sehr fest wird. In der DE-PS 197 23 538 werden breiartige Zweikomponenten-Zubereitungen zur Anfertigung einer auftragefähigen, breiartigen Zubereitung zum Bleichen von Humanhaaren beschrieben, welche neben den üblichen Bestandteilen von Blondierungen eine bestimmte Verdickerkombination, bestehend aus einem Acrylsäurepolymer und mindestens einem Polymer aus der Gruppe der Cellulosen, Alginate und Polysaccharide, mindestens einem Mineralöl, mindestens einem flüssigen, langkettigen, hydrophoben Fettsäureester und mindestens einem wachsförmigen, langkettigen, hydrophoben Fettsäureester und/oder synthetischen Bienenwachsersatzstoff enthalten. Diese Mittel sind jedoch in Bezug auf ihr Verhalten bei hohen Temperaturen nicht in jeder Hinsicht befriedigend. In der DE-OS 199 09 661 wird die Verwendung einer speziellen Blondiermittelsuspension auf der Basis von Lipogele oder Oleogele ausbildenden Bestandteilen beschrieben.

Es bestand jedoch weiterhin ein Bedarf für ein lagerstabiles, nichtstaubendes Mittel zum Entfärben oder Blondieren von Haaren, welches vor Gebrauch durch einfaches Schütteln oder Anrühren mit einer Oxidationsmittelzubereitung vermischt wird und neben seiner absolut staubfreien Darreichungs- und Anwendungsform eine sehr gute Blondierwirkung bei gleichzeitiger guter Lagerstabilität zwischen 5 und 45 °C gewährleistet, ohne die hervorragende, cremeartige Konsistenz und damit die hervorragenden Gebrauchseigenschaften unter Kälteeinfluß bzw. Wärmeeinfluß zu verlieren.

Überraschend wurde nunmehr gefunden, dass die vorstehend beschriebene Aufgabe durch Verwendung eines pastenförmigen Blondiermittels basierend auf einer speziellen, neuartigen Kombination von Verdickungsmitteln gelöst werden kann, wobei der Zusatz von Emulgatoren nicht erforderlich ist.

Gegenstand der vorliegenden Erfindung ist daher ein Mittel zum Entfärben oder Blondieren von Haaren, insbesondere menschlichen Haaren, welches unmittelbar vor der Anwendung mit einer wässrigen Oxidationsmittelzubereitung vermischt wird und dadurch gekennzeichnet ist, dass es in Form einer Blondiermittelsuspension vorliegt und eine Kombination aus
(a) 0,1 bis 80 Gewichtsprozent mindestens einer organischen lipophilen Verbindung aus der Gruppe der pflanzlichen und tierischen Fette, Öle und Wachse, der Paraffinkohlenwasserstoffe, der höheren Alkohole und Ether, der aliphatischen und aromatischen Ester sowie der Silikonöle;
(b) 0,01 bis 20 mindestens eines anorganischen oder organischen Verdickers mit lipophilem Charakter aus der Gruppe der Bentonite (hydratisierte kolloidale Aluminiumsilkat-Tone und deren Derivate) und der Dextrinpalmitinsäureester (Dextrin Palmitate);
(c) 0,1 bis 40 Gewichtsprozent mindestens eines anorganischen oder organischen Verdickers mit hydrophilem Charakter;
(d) 10 bis 65 Gewichtsprozent mindestens eines anorganischen Persalzes;
(e) 10 bis 45 Gewichtsprozent mindestens eines alkalisch reagierenden Salzes;
sowie gegebenenfalls Hilfsstoffen und Zusatzstoffen enthält.

Als organisch lipophile Verbindungen eignen sich insbesondere Pflanzenöle wie zum Beispiel Jojobaöl; Vaseline; flüssige Paraffine, beispielsweise Paraffinum Perliquidum und Paraffinum Subliquidum; Siliconöle; flüssige, langkettige, hydrophobe Fettsäureester, beispielsweise Octylpalmitat, Isocetylpalmitat, Isopropylpalmitat und Octylstearat; wachsförmige, langkettige, hydrophobe Fettsäureester und/oder synthetische Wachsersatzstoffe, beispielsweise natürliches oder synthetisches Bienenwachs (zum Beispiel Lipowachs 6138G® der Firma Lipo Chemicals), C18- bis C36-Fettsäuren (zum Beispiel Synchrowachs AW1C® der Firma Croda Chemicals Ltd.), C-10 bis C36-Fettsäuretriglyceride, wie zum Beispiel Octansäure/Dodecansäure-Triglycerid, hydrierte Kokosfettsäureglyceride (zum Beispiel Softisan 100® der Firma Hüls AG), Glyceryltribehenat (beispielsweise Synchrowachs HRC® der Firma Croda Chemicals Ltd.), Fettsäureestergemische (beispielsweise Cutina BW® der Firma Henkel KGaA), sowie deren Mischungen. Besonders bevorzugt ist hierbei die Verwendung von Jojobaöl, Fettsäureestern, Paraffinöl, Kombinationen aus Fettsäureestern und Paraffinöl sowie Kombinationen aus Fettsäureestern und/oder Paraffinöl mit Vaseline.

Die lipophilen Verbindungen werden, bezogen auf die Gesamtmenge der Blondiermittelsuspension, in einer Gesamtmenge von etwa 0,1 bis 80 Gewichtsprozent, vorzugsweise von 3 bis 65 Gewichtsprozent, und insbesondere von 20 bis 50 Gewichtsprozent, eingesetzt.

Als anorganischer oder organischer Verdicker mit lipophilem Charakter sind insbesondere Quaternium-18 Bentonite (beispielsweise Tixogel MP 100 der Fa. Südchemie) und Dextrin Palmitate (beispielsweise Rheopearl KL der Chiba Flour Milling Co., Ltd.) sowie Mischungen dieser Verbindungen zu nennen.

Die anorganischen oder organischen Verdicker mit lipophilem Charakter oder deren Gemische bilden bei Auflösung in den beschriebenen lipophilen Verbindungen ein Oleogel beziehungsweise Lipogel aus. Das Auflösen des lipophilen Verdickers in der lipophilen Komponente kann hierbei durch Erhitzen oder durch die Verwendung von Lösungsvermittlern, wie zum Beispiel Propylencarbonat, unterstützt werden.

Bezogen auf die Gesamtmenge der Blondiermittelsuspension werden die anorganischen oder organischen Verdicker mit lipophilem Charakter in einer Gesamtmenge von etwa 0,1 bis 40 Gewichtsprozent, vorzugsweise von 0,1 bis 10 Gewichtsprozent eingesetzt.

Als hydrophile anorganische oder organische Verdicker können Polymere aus der Gruppe der Cellulosen, Alginate, Polysaccharide und Acrylsäuren, vorzugsweise Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum oder Xanthan Gum, oder Acrylsäurepolymere mit einem Molekulargewicht von etwa 1.250.000 bis 4.000.000, alleine oder in Kombination miteinander, verwendet werden, wobei die Verwendung von anquellverzögernden Methylhydroxyethylcellulosen, einer Kombination von Alginaten mit Polysacchariden oder/und Cellulosen oder einer Kombination von Alginaten und/oder Cellulosen mit Acrylsäurepolymeren besonders bevorzugt ist.

Bezogen auf die Gesamtmenge der Blondiermittelsuspension werden die hydrophilen Verdicker in einer Gesamtmenge von etwa 0,1 bis 40 Gewichtsprozent, vorzugsweise von 0,2 bis 20 Gewichtsprozent, und insbesondere von 0,5 bis 15 Gewichtsprozent eingesetzt.

Als anorganische Persalze werden vorzugsweise Erdalkaliperoxide und anorganische Persulfate, beispielsweise Ammoniumpersulfat und Alkalipersulfate wie Natriumpersulfat oder Kaliumpersulfat, oder Mischungen dieser anorganischen Persalze eingesetzt, wobei die Persalze bezogen auf die Gesamtmenge der Blondiermittelsuspension in einer Gesamtmenge von vorzugsweise 10 bis 65 Gewichtsprozent, insbesondere von 20 bis 55 Gewichtsprozent, eingesetzt werden.

Als alkalisch reagierende Salze werden in wässriger Lösung alkalisch reagierende Alkalimetallsalze oder Erdalkalimetallsalze, beispielsweise Natriumcarbonat, Natriumhydrogencarbonat, Magnesiumcarbonat, Ammoniumcarbonat, Ammoniumhydrogencarbonat, Natriumsilikate oder Mischungen dieser Salze, verwendet, wobei diese Salze bezogen auf die Gesamtmenge der Blondiermittelsuspension in einer Gesamtmenge von vorzugsweise 10 bis 45 Gewichtsprozent, insbesondere von 15 bis 35 Gewichtsprozent, eingesetzt werden.

Zusätzlich kann die cremeförmige Blondiermittelsuspension weitere für derartige Zubereitungen übliche Zusatzstoffe, wie zum Beispiel Pflegestoffe, Siliciumdioxid, Titandioxid, Chelatisierungsmittel für Schwermetallionen, beispielsweise Ethylendiamintetraessigsäure, Anfärbestoffe, beispielsweise Ultramarinfarbstoffe, oder Parfüme, enthalten, wobei diese Zusatzstoffe in den für solche Mittel üblichen Einsatzmengen eingesetzt werden; beispielsweise die Pflegestoffe, das Siliciumdioxid und das Chelatisierungsmittel jeweils in einer Menge von 0,01 bis 3 Gewichtsprozent, und die Anfärbestoffe und Parfüme jeweils in einer Menge von 0,01 bis 2 Gewichtsprozent.

Ebenfalls ist es möglich, dass die erfindungsgemäße Blondiermittelsuspension neben den vorgenannten anorganischen oder organischen Verdickern der Komponente b) zusätzlich lipophile anorganische oder organische Verdicker wie zum Beispiel Alkalicarboxylate, Erdalkalicarboxylate oder Aluminiumcarboxylate, vorzugsweise Natriumpalmitat, Aluminium/Magnesiumhydroxystearat oder Magnesiumstearat, Aluminiummonostearat, Aluminiummonodistearat und/oder Aluminiumtristearat; Copolymerisate von Alkenen, vorzugsweise Beispiel Ethylen/Propylen-Copolymere; vemetzte organische Polymere und lipophilisierte Schichtsilikate, vorzugsweise Benzyl-dimethylstearylammonium-Hectorit (beispielsweise Bentone 28 der Fa. Rheox), sowie Gemische dieser lipophilen Verdicker enthalten, wobei der Zusatz von Alkalistearaten, Erdalkalistearaten, Aluminiumstearaten und Aluminium/Magnesiumhydroxystearaten, und insbesondere Magnesiumstearaten und Aluminiumstearaten, bevorzugt ist. Die Einsatzmenge der zusätzlichen lipophilen anorganischen oder organischen Verdicker beträgt vorzugsweise 0,2 bis 20 Gewichtsprozent. Ebenfalls ist es möglich, in bestimmten Fällen fertige Gemische dieser lipophilen Verdicker mit den lipophilen Verbindungen der Komponente a), beispielsweise Brooks Gel® der Fa. Brooks Industries, die Bentone Gel®-Typen der Fa. Rheox, die Myglyol Gel®- und Softisan Gel®-Typen der Fa. Hüls AG sowie die Gilugel®-Typen der Fa. BK Giulini Chemie, zu verwenden.

Vorzugsweise enthält die erfindungsgemäße Blondiermittelsuspension keine Tenside oder Emulgatoren und ist wasserfrei, wobei ein Wassergehalt von maximal bis zu 3 Gewichtsprozent jedoch zulässig ist.

Vor der Anwendung wird die erfindungsgemäße Blondiermittelsuspension mit der Oxidationsmittelzubereitung zu einem auftragefähigen Blondierbrei vermischt, wobei dieses Vermischen in einer Schale oder durch Anschütteln in einer Auftrageflasche erfolgen kann. Als Oxidationsmittelzubereitung kann insbesondere eine Wasserstoffperoxid enthaltende wässrige Lösung oder Öl-in-Wasser-Emulsion (insbesondere eine 6- bis 12-prozentige Wasserstoffperoxidlösung oder Wasserstoffperoxidemulsion) verwendet werden. Es ist jedoch auch möglich, anstelle von Wasserstoffperoxid Wasserstoffperoxid-abspaltende Addukte, beispielsweise Harnstoffperoxid oder Melaminperhydrat, zu verwenden.

Das Mischungsverhältnis von Blondiermittelsuspension zu Oxidationsmittelzubereitung beträgt vorzugsweise 2:1 bis 1:8, insbesondere 1:1 bis 1:5.

Das nach dem Vermischen mit der Oxidationsmittelzubereitung erhaltene gebrauchsfertige Mittel zum Entfärben oder Blondieren von Haaren weist einen pH-Wert von etwa 7,5 bis 11, insbesondere von 8 bis 9,5, auf.

Das gebrauchsfertige Mittel wird auf das Haar gleichmäßig aufgetragen und nach einer Einwirkungszeit von 15 bis 60 Minuten bei Raumtemperatur (20-25°C) beziehungsweise von 10 bis 50 Minuten bei Wäremeinwirkung (30-50°C) mit Wasser ausgespült.

Die cremeförmige Blondiermittelsuspension kann je nach ihrer Viskosität in Tuben, Sachets oder Tiegel abgefüllt werden. Neben der anwendungsfreundlichen Produktviskosität über einen großen Temperaturbereich und der leichten Anmischbarkeit mit dem Oxidationsmittel zeichnet sich das erfindungsgemäße Mittel durch eine hervorragende Lagerstabilität, Auftragefähigkeit, Verteilbarkeit und Haftung am Haar sowie ein breites Anwendungsspektrum aus. Im Vergleich zu herkömmlichen Blondiermitteln erreicht das erfindungsgemäße Mittel eine bessere Aufhellungen bei geringerem Gehalt an aktiven Bleichwirkstoffen. Durch die emulgierenden Eigenschaften der gelbildenden Komponenten läßt sich das erfindungsgemäße Blondiermittel sehr leicht mit Wasser wieder rückstandsfrei aus dem Haar ausspülen.

Die nachfolgenden Beispiele sollen den Gegenstand näher erläutern, ohne diesen hierauf zu beschränken.

### Beispiele

### Beispiel 1: Blondiermittel

| Cremeförmige Blondiermittelsuspension | |
|---|---|
| 8,0 g | Isododecan/Ethylen-Mixed-Copolymer (Brooks Gel® der Brooks Industries Inc.) |
| 8,0 g | Isopropylpalmitat |
| 16,0 g | Jojobaöl |
| 4,0 g | C10-C18 Fettsäure-Triglycerid (Nesatol® der Fa. Vevy) |
| 24,2 g | Natriummetasilikat |
| 4,0 g | Natriumalginat |
| 11,3 g | Diammoniumpersulfat |
| 22,5 g | Dikaliumpersulfat |
| 1,0 g | Dextrin Palmitate (Rheopearl KL der Chiba Flour Milling Co.) |
| 1,0 g | Ethylendiamintetraessigsäure-Dinatriumsalz |
| 100,0 g | |

Zur Herstellung der cremeförmigen Blondiermittelsuspension werden die flüssigen Komponenten zunächst auf 75 bis 80 °C erhitzt und mit dem Dextrin Palmitat bei 80 °C homogen vermischt. Anschließend werden die vorgemischten festen Rohstoffe eingetragen, wobei darauf geachtet wird, dass eine homogene Verteilung der Feststoffe in der Lipogelmatrix erfolgt.

### Anwendung

25 g der vorstehend beschriebenen Blondiermittelsuspension 1 werden mit 75 g einer 6prozentigen wässrigen Wasserstoffperoxidlösung der folgenden Zusammensetzung

| | |
|---|---|
| 6,0 g | Wasserstoffperoxid |
| 2,0 g | Cetylstearylalkohol |
| 0,2 g | Lanolinalkohol |
| 0,1 g | Phosphorsäure (85prozentig) |
| 91,7 g | Wasser |
| 100,0 g | |

In einer Auftrageflasche 10 bis 15 Sekunden geschüttelt. Anschließend wird das Blondiermittel mittels einer Auftrageflasche gleichmäßig auf das aufzuhellende Haar aufgetragen. Nach einer Einwirkungszeit von 30 Minuten bei Raumtemperatur (20 bis 30° C) wird das Haar mit warmem Wasser gründlich ausgespült und getrocknet.

Der Aufhellungsgrad beträgt 3 Tonstufen und kann durch Verlängerung der Einwirkungszeit um 20 Minuten um 1 bis 2 Tonstufen erhöht werden.

### Beispiel 2: Blondiermittel

| Cremeförmige Blondiermittelsuspension | |
|---|---|
| 1,9 g | Quaternium-18 Bentonite (Tixogel MP 100 der Fa. Südchemie) |
| 34,1 g | Paraffinöl |
| 23,0 g | Dinatriumpersulfat |
| 17,0 g | Dikaliumpersulfat |
| 20,0 g | Natriummetasilikat |
| 1,5 g | Xanthan Gum |
| 1,5 g | Acrylsäurepolymer (Synthalen® K der Fa. 3V-Sigma) |
| 0,5 g | Ethylendiamintetraessigsäure-Dinatriumsalz |
| 100,0 g | |

Zur Herstellung dieser cremeförmigen Blondiermittelsuspension wird das Quaternium-18 Bentonite dem Paraffinöl zugegeben und das Gemisch anschließend auf 75 °C erhitzt und 10 Minuten lang mit einem Rotor-Stator-System bei 15.000 U/min bei Raumtemperatur homogenisiert. Die vorgemischten festen Rohstoffe werden anschließend in das auf diese Weise hergestellte Lipogel eingetragen, wobei darauf geachtet wird, dass eine homogene Verteilung der Feststoffe in der Lipogelmatrix erfolgt.

### Anwendung

25 g der vorstehenden Blondiermittelsuspension werden mit 25 g einer 9prozentigen wasserstoffperoxidhaltigen Öl-in-Wasser-Emulsion der folgenden Zusammensetzung

| | |
|---|---|
| 9,0 g | Wasserstoffperoxid |
| 2,0 g | Cetylstearylalkohol |
| 0,2 g | Lanolinalkohol |
| 0,1 g | Phosphorsäure (85-prozentig) |
| 88,7 g | Wasser |
| 100,0 g | |

in einer Schale mit einem Pinsel homogen verrührt.

Das so erhaltene, breiartige Blondiermittel wird auf mittelbraunes Haar gleichmäßig aufgetragen und nach einer Einwirkungszeit von 30 Minuten bei Raumtemperatur mit warmem Wasser abgespült und getrocknet. Das so behandelte Haar ist auf einen hellblonden Farbton aufgehellt worden.

### Beispiel 3: Blondiermittel

| Cremeförmige Blondiermittelsuspension | |
|---|---|
| 41,5 g | Octylstearat |
| 2,5 g | Paraffin Perliquidum |
| 2,0 g | Vaseline |
| 2,0 g | Dextrin Palmitate (Rheopearl KL der Chiba Flour Milling Co.) |
| 2,0 g | Jojobaöl |
| 4,0 g | Dinatriumpersulfat |
| 17,0 g | Dikaliumpersulfat |
| 8,0 g | Diammoniumpersulfat |
| 18,0 g | Natriummetasilikat |
| 2,5 g | Natriumalginat |
| 0,5 g | Ethylendiamintetraessigsäure-Dinatriumsalz |
| 100,0 g | |

Zur Herstellung dieser cremeförmigen Blondiermittelsuspension wird das Dextrin Palmitat im lipophilen Gemisch Octylstearat, Paraffinöl und Vaseline unter Erhitzen auf 90°C vollständig aufgelöst. Das Jojobaöl wird bei 70°C homogen im abkühlenden Oleogel verteilt. Die vorgemischten festen Rohstoffe werden anschließend in das nach Abkühlen auf Raumtemperatur entstandene Lipogel eingetragen, wobei darauf geachtet wird, dass eine homogene Verteilung der Feststoffe in der Lipogelmatrix erfolgt.

### Anwendung

25 g der vorstehend beschriebenen Blondiermittelsuspension werden mit 37,5 g einer 6prozentigen wässrigen Wasserstoffperoxidlösung in einer Schale mit einem Pinsel homogen verrührt. Es ist jedoch auch möglich, die Wasserstoffperoxidlösung in einer Auftrageflasche vorzulegen und mit der Blondiermittelsuspension zum gebrauchsfertigen Blondiermittel anzuschütteln. Das Blondiermittel wird gleichmäßig auf das aufzuhellende Haar aufgetragen und nach einer Einwirkungszeit von 40 Minuten bei Raumtemperatur mit Wasser abgespült. Sodann wird das Haar getrocknet. Der Aufhellungsgrad beträgt etwa 4 Tonstufen.

Alle Prozentangaben stellen, soweit nicht anders angegeben, Gewichtsprozente dar.

## Patentansprüche

1. Mittel zum Entfärben oder Blondieren von Haaren, welches unmittelbar vor der Anwendung mit einer wässrigen Oxidationsmittelzubereitung vermischt wird und **dadurch gekennzeichnet ist, dass** es in Form einer Blondiermittelsuspension vorliegt und eine Kombination aus
(a) 0,1 bis 80 Gewichtsprozent mindestens einer organisch lipophilen Verbindung aus der Gruppe der pflanzlichen und tierischen Fette, Öle und Wachse, der Paraffinkohlenwasserstoffe, der höheren Alkohole und Ether, der aliphatischen und aromatischen Ester sowie der Silikonöle;
(b) 0,01 bis 20 mindestens eines anorganischen oder organischen Verdickers mit lipophilem Charakter aus der Gruppe der Bentonite und der Dextrinpalmitinsäureester;
(c) 0,1 bis 40 Gewichtsprozent mindestens eines anorganischen oder organischen Verdickers mit hydrophilem Charakter,
(d) 10 bis 65 Gewichtsprozent mindestens eines anorganischen Persalzes,
(e) 10 bis 45 Gewichtsprozent mindestens eines alkalisch reagierenden Salzes,
sowie gegebenenfalls Hilfsstoffen und Zusatzstoffen enthält.

2. Mittel nach Anspruch 1, **dadurch gekennzeichnet, dass** die organisch lipophile Verbindung ausgewählt ist aus Pflanzenölen; Vaseline; flüssigen Paraffinen; Siliconölen; flüssigen, langkettigen, hydrophoben Fettsäureestem; natürlichem oder synthetischem Bienenwachs; C18- bis C36-Fettsäuren; C-10 bis C36-Fettsäuretriglyceriden und Fettsäureestergemischen oder Mischungen dieser Verbindungen.

3. Mittel nach Anspruch 2, **dadurch gekennzeichnet, dass** die organisch lipophile Verbindung ausgewählt ist aus Jojobaöl, Fettsäureestern, Paraffinöl, Kombinationen aus Fettsäureestern und Paraffinöl sowie Kombinationen aus Fettsäureestern und/oder Paraffinöl mit Vaseline.

4. Mittel nach einem der Ansprüche 1 bis 3, **dadurch gekennzeichnet, dass** der lipophile anorganische oder organische Verdicker ausgewählt ist aus Quaternium-18 Bentonite und Dextrin Palmitate oder Mischungen dieser Verdicker.

5. Mittel nach einem der Ansprüche 1 bis 4, **dadurch gekennzeichnet, dass** der hydrophile anorganische oder organische Verdicker ausgewählt ist aus Polymeren aus der Gruppe der Cellulosen, Alginate, Polysaccharide und Acrylsäuren.

6. Mittel nach Anspruch 5, **dadurch gekennzeichnet, dass** der hydrophile anorganische oder organische Verdicker ausgewählt ist aus Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum, Xanthan Gum und Acrylsäurepolymeren mit einem Molekulargewicht von etwa 1.250.000 bis 4.000.000, oder Mischungen dieser Verdicker.

7. Mittel nach einem der Ansprüche 1 bis 6, **dadurch gekennzeichnet, dass** das anorganische Persalz ausgewählt ist aus Erdalkaliperoxiden, Ammoniumpersulfaten und Alkalipersulfaten.

8. Mittel nach einem der Ansprüche 1 bis 7, **dadurch gekennzeichnet, dass** das alkalisch reagierende Salz ausgewählt ist aus Natriumcarbonat, Natriumhydrogencarbonat, Magnesiumcarbonat, Ammoniumcarbonat, Ammoniumhydrogencarbonat und Natriumsilikaten oder Mischungen dieser Salze.

9. Mittel nach einem der Ansprüche 1 bis 8, **dadurch gekennzeichnet, dass** es zusätzlich einen oder mehrere lipophile anorganische oder organische Verdicker aus der Gruppe der Alkalicarboxylate, Erdalkalicarboxylate oder Aluminiumcarboxylate, Copolymerisate von Alkenen, vernetzten organischen Polymere und lipophilisierten Schichtsilikate enthält.

10. Mittel nach einem der Ansprüche 1 bis 9, **dadurch gekennzeichnet, dass** es frei von Tensiden ist.

11. Mittel nach einem der Ansprüche 1 bis 10, **dadurch gekennzeichnet, dass** als wässrige Oxidationsmittelzubereitung eine 6- bis 12prozentige wässrige Wasserstoffperoxidlösung oder Wasserstoffperoxidemulsion verwendet wird.

12. Mittel nach einem der Ansprüche 1 bis 11, **dadurch gekennzeichnet, dass** das Mischungsverhältnis von Blondiermittelsuspension zu Oxidationsmittelzubereitung 2:1 bis 1:8 beträgt.

13. Mittel nach einem der Ansprüche 1 bis 12, **dadurch gekennzeichnet, dass** das gebrauchsfertige Mittel zum Entfärben oder Blondieren von Haaren einen pH-Wert von 7,5 bis 11 aufweist.

## Claims

1. Agent for decolouring or bleaching hair, which is mixed directly prior to application with an aqueous oxidizing agent preparation and is **characterized in that** it is in the form of a bleaching agent suspension and comprises a combination of
(a) 0.1 to 80 per cent by weight of at least one organic-lipophilic compound from the group of vegetable and animal fats, oils and waxes, of paraffin hydrocarbons, of higher alcohols and ethers, of aliphatic and aromatic esters, and of silicone oils;
(b) 0.01 to 20 per cent by weight of at least one inorganic or organic thickener with lipophilic character from the group of bentonites and of dextrin palmitic esters;
(c) 0.1 to 40 per cent by weight of at least one inorganic or organic thickener with hydrophilic character,
(d) 10 to 65 per cent by weight of at least one inorganic persalt,
(e) 10 to 45 per cent by weight of at least one alkaline-reacting salt,
and optionally auxiliaries and additives.

2. Agent according to Claim 1, **characterized in that** the organic-lipophilic compound is chosen from plant oils; petroleum jelly; liquid paraffin; silicone oils; liquid, long-chain, hydrophobic fatty acid esters; natural or synthetic beeswax; C18- to C36-fatty acids; C10- to C36-fatty acid triglycerides and fatty acid ester mixtures or mixtures of these compounds.

3. Agent according to Claim 2, **characterized in that** the organic-lipophilic compound is chosen from jojoba oil, fatty acid esters, paraffin oil, combinations of fatty acid esters and paraffin oil, and combinations of fatty acid esters and/or paraffin oil with petroleum jelly.

4. Agent according to one of Claims 1 to 3, **characterized in that** the lipophilic inorganic or organic thickener is chosen from quaternium-18 bentonite and dextrin palmitate or mixtures of these thickeners.

5. Agent according to one of Claims 1 to 4, **characterized in that** the hydrophilic inorganic or organic thickener is chosen from polymers from the group of celluloses, alginates, polysaccharides and acrylic acids.

6. Agent according to Claim 5, **characterized in that** the hydrophilic inorganic or organic thickener is chosen from methylcelluloses, ethylcelluloses, hydroxyethylcelluloses, methylhydroxyethylcelluloses, methylhydroxypropylcelluloses, carboxymethylcelluloses, alginic acid, sodium alginate, ammonium alginate, calcium alginate, gum arabic, guar gum, xanthan gum and acrylic acid polymers with a molecular weight of from about 1 250 000 to 4 000 000, or mixtures of these thickeners.

7. Agent according to one of Claims 1 to 6, **characterized in that** the inorganic persalt is chosen from alkaline earth metal peroxides, ammonium persulphates and alkali metal persulphates.

8. Agent according to one of Claims 1 to 7, **characterized in that** the alkaline-reacting salt is chosen from sodium carbonate, sodium hydrogencarbonate, magnesium carbonate, ammonium carbonate, ammonium hydrogencarbonate and sodium silicates or mixtures of these salts.

9. Agent according to one of Claims 1 to 8, **characterized in that** it additionally comprises one or more lipophilic inorganic or organic thickeners from the group of alkali metal carboxylates, alkaline earth metal carboxylates or aluminium carboxylates, copolymers of alkenes, crosslinked organic polymers and lipophilized phyllosilicates.

10. Agent according to one of Claims 1 to 9, **characterized in that** it is free from surfactants.

11. Agent according to one of Claims 1 to 10, **characterized in that** the aqueous oxidizing agent preparation used is a 6 to 12 per cent strength aqueous hydrogen peroxide solution or hydrogen peroxide emulsion.

12. Agent according to one of Claims 1 to 11, **characterized in that** the mixing ratio of bleaching agent suspension to oxidizing agent preparation is 2:1 to 1:8.

13. Agent according to one of Claims 1 to 12, **characterized in that** the ready-to-use agent for decolouring or bleaching hair has a pH of from 7.5 to 11.

## Revendications

1. Agent destiné à décolorer ou colorer en blond les cheveux, qui est mélangé immédiatement avant l'emploi avec une préparation aqueuse d'oxydant et est **caractérisé en ce qu'**il se trouve sous forme d'une suspension d'agent de coloration en blond et contient une association de
(a) 0,1 à 80 % en poids d'au moins un composé organique lipophile choisi dans le groupe des graisses, huiles et cires animales et végétales, des hydrocarbures paraffiniques, des alcools et éthers supérieurs, des esters aliphatiques et aromatiques ainsi que des huiles de silicone ;
(b) 0,01 à 20 % en poids d'au moins un épaississant organique ou minéral à caractère lipophile, choisi dans le groupe des bentonites et des palmitates de dextrine ;
(c) 0,1 à 40 % en poids d'au moins un épaississant organique ou minéral à caractère lipophile ;
(d) 10 à 65 % en poids d'au moins un persel minéral ;
(e) 10 à 45 % en poids d'au moins un sel à réaction alcaline ;
ainsi qu'éventuellement des additifs et adjuvants.

2. Agent selon la revendication 1, **caractérisé en ce que** le composé organique lipophile est choisi parmi les huiles minérales ; la vaseline ; les paraffines liquides ; les huiles de silicone ; les esters d'acides gras liquides, à longue chaîne, hydrophobes ; la cire d'abeille naturelle ou synthétique ; les acides gras en C₁₈-C₃₆ ; les triglycérides d'acides gras en C₁₀-C₃₆ et des mélanges d'esters d'acides gras ou des mélanges de ces composés.

3. Agent selon la revendication 2, **caractérisé en ce que** le composé organique lipophile est choisi parmi l'huile de jojoba, des esters d'acides gras, l'huile de paraffine, des associations d'esters d'acides gras et d'huile de paraffine ainsi que des associations d'esters d'acides gras et/ou d'huile de paraffine avec la vaseline.

4. Agent selon l'une quelconque des revendications 1 à 3, **caractérisé en ce que** l'épaississant organique ou minéral lipophile est choisi parmi la quaternium-18 bentonite et le palmitate de dextrine ou des mélanges de ces épaississants.

5. Agent selon l'une quelconque des revendications 1 à 4, **caractérisé en ce que** l'épaississant organique ou minéral hydrophile est choisi parmi des polymères du groupe des celluloses, alginates, polysaccharides et acides acryliques.

6. Agent selon la revendication 5, **caractérisé en ce que** l'épaississant organique ou minéral hydrophile est choisi parmi les méthylcelluloses, les éthylcelluloses, les hydroxyéthylcelluloses, les méthylhydroxyéthylcelluloses, les méthylhydroxypropylcelluloses, les carboxyméthylcelluloses, l'acide alginique, l'alginate de sodium, l'alginate d'ammonium, l'alginate de calcium, la gomme arabique, la gomme guar, la gomme xanthane et les polymères d'acide acrylique ayant une masse moléculaire d'environ 1 250 000 à 4 000 000, ou des mélanges de ces épaississants.

7. Agent selon l'une quelconque des revendications 1 à 6, **caractérisé en ce que** le persel minéral est choisi parmi les peroxydes de métaux alcalino-terreux, les persulfates d'ammonium et les persulfates de métaux alcalins.

8. Agent selon l'une quelconque des revendications 1 à 7, **caractérisé en ce que** le sel à réaction alcaline est choisi parmi le carbonate de sodium, l'hydrogénocarbonate de sodium, le carbonate de magnésium, le carbonate d'ammonium, l'hydrogénocarbonate d'ammonium et les silicates de sodium ou des mélanges de ces sels.

9. Agent selon l'une quelconque des revendications 1 à 8, **caractérisé en ce qu'**il contient en outre un ou plusieurs épaississants organiques ou minéraux lipophiles choisis dans le groupe des carboxylates de métaux alcalins, carboxylates de métaux alcalino-terreux et carboxylates d'aluminium, des copolymères d'alcènes, des polymères organiques réticulés et des silicates lamellaires rendus lipophiles.

10. Agent selon l'une quelconque des revendications 1 à 9, **caractérisé en ce qu'**il est exempt de tensioactifs.

11. Agent selon l'une quelconque des revendications 1 à 10, **caractérisé en ce qu'**on utilise en tant que préparation aqueuse d'oxydant une solution aqueuse de peroxyde d'hydrogène ou émulsion aqueuse de peroxyde d'hydrogène à 6-12 %.

12. Agent selon l'une quelconque des revendications 1 à 11, **caractérisé en ce que** le rapport de mélange de la suspension d'agent de coloration en blond à la préparation d'oxydant va de 2:1 à 1:8.

13. Agent selon l'une quelconque des revendications 1 à 12, **caractérisé en ce que** l'agent pour la décoloration ou la coloration en blond des cheveux, prêt à l'emploi, présente un pH de 7,5 à 11.
